# EUROPÄISCHE PATENTANMELDUNG

(11) **EP 2 495 597 A1**
(43) Veröffentlichungstag der Anmeldung: **05.09.2012**
(21) Anmeldenummer: 11156350.8
(22) Anmeldetag: 01.03.2011
(51) Int. Cl.: G02B 21/00, G02B 21/22

(54) **Operationsmikroskop mit zwei Vergrößerungssystemen**

(71) Anmelder: Möller-Wedel GmbH, 22871 Wedel (DE)
(72) Erfinder: Bielor, Rolf-Dieter, 22880 Wedel (DE)
(74) Vertreter: Glawe, Delfs, Moll

(57) **Zusammenfassung**

Die Erfindung betrifft ein Operationsmikroskop mit einer Frontlinse (14), einem Einblick (17) und einem zwischen der Frontlinse (14) und dem Einblick (17) angeordneten ersten Vergrößerungssystem (15). Die Frontlinse (14), das erste Vergrößerungssystem (15) und der Einblick (17) definieren einen Beobachtungsstrahlengang (20A, 20B). Das erste Vergrößerungssystem (15) hat einen variablen Vergrößerungsfaktor. Erfindungsgemäß ist zwischen der Frontlinse (14) und dem Einblick (17) ein zweites Vergrößerungssystem (16) angeordnet ist. Das erfindungsgemäße Operationsmikroskop ermöglicht einen großen Verstellbereich der Vergrößerungsfaktors bei hoher optischer Qualität.

## Beschreibung

Die Erfindung betrifft ein Operationsmikroskop, das eine Frontlinse, einen Einblick und ein zwischen der Frontlinse und dem Einblick angeordnetes erstes afokales Vergrößerungssystem umfasst. Die Frontlinse, das erste Vergrößerungssystem und der Einblick definieren einen Beobachtungsstrahlengang. Das erste Vergrößerungssystem hat einen variablen Vergrößerungsfaktor.

Solche Operationsmikroskope sind in verbreiteter Verwendung und dienen dazu, dem Chirurgen bei einer Operation eine vergrößerte Ansicht des Operationsfelds zu liefern. Die Frontlinse des Operationsmikroskops wird in passendem Abstand zum Operationsfeld angeordnet und der Chirurg sieht in einem Okular des Einblicks das vergrößerte Bild. Damit das Operationsmikroskop an die jeweiligen Gegebenheiten angepasst werden kann, hat das Vergrößerungssystem einen variablen Vergrößerungsfaktor. Bei hohem Vergrößerungsfaktor können Details im Operationsfeld in hoher Auflösung betrachtet werden. Bei niedrigem Vergrößerungsfaktor verschafft der Chirurg sich einen Überblick über das Operationsfeld.

Es hat sich gezeigt, dass die mit den heute gängigen Operationsmikroskopen mögliche Vergrößerung den Anforderungen nicht immer genügt. Mit den zur Verfügung stehenden Hilfsmitteln können Operationen in immer kleinerem Maßstab und mit immer höherer Genauigkeit durchgeführt werden. Operationsmikroskope sollen deswegen eine hohe Vergrößerung erlauben, während andererseits die Möglichkeit, das Operationsfeld im Überblick zu betrachten, nicht verloren gehen soll.

Der Erfindung liegt die Aufgabe zu Grunde, ein Operationsmikroskop vorzustellen, das flexibler eingesetzt werden kann. Ausgehend vom eingangs genannten Stand der Technik wird die Aufgabe gelöst mit den Merkmalen des Anspruchs 1. Erfindungsgemäß ist zwischen der Frontlinse und dem Einblick ein zweites afokales Vergrößerungssystem angeordnet. Vorteilhafte Ausführungsformen finden sich in den Unteransprüchen.

Zunächst werden einige Begriffe erläutert. Ein afokales Vergrößerungssystem bezeichnet eine Einheit von optischen Elementen, die so angeordnet sind, dass zwei Lichtstrahlen, die parallel zueinander und parallel zur optischen Achse des Vergrößerungssystems in das Vergrößerungssystem eintreten, auch parallel wieder austreten, wobei der Abstand zwischen den Lichtstrahlen beim Eintritt ein anderer ist als beim Austritt. In dem Vergrößerungssystem wird regelmäßig kein Zwischenbild erzeugt. Auch eine Bildumkehr findet in dem Vergrößerungssystem regelmäßig nicht statt.

Wenn der Vergrößerungsfaktor eines Vergrößerungssystems verändert wird, verändert sich bei gleich bleibendem Abstand der eintretenden Lichtstrahlen der Abstand der austretenden Lichtstrahlen voneinander. Ein Vergrößerungssystem kann sowohl den Fall umfassen, dass der Abstand der eintretenden Lichtstrahlen größer ist als der Abstand der austretenden Lichtstrahlen, als auch den Fall, dass der Abstand der eintretenden Lichtstrahlen kleiner ist als der Abstand der austretenden Lichtstrahlen. Bei einem variablen Vergrößerungsfaktor kann als Sonderfall umfasst sein, dass der Abstand der eintretenden Lichtstrahlen und der austretenden Lichtstrahlen übereinstimmt, also keine Vergrößerung oder Verkleinerung stattfindet. Der Begriff Dehnungsfaktor bezeichnet den Quotienten aus dem größtmöglichen und dem kleinstmöglichen Vergrößerungsfaktor.

Ein Einblick ist eine Einheit von optischen Elementen, die so angeordnet sind, dass aus parallel eintretenden Lichtstrahlen eine Abbildung erzeugt werden kann, so dass die Abbildung durch ein Okular betrachtet werden kann. In dem Operationsmikroskop sind die Frontlinse, die Vergrößerungssysteme und der Einblick so angeordnet, dass in dem Okular eine vergrößerte Abbildung eines vor der Frontlinse angeordneten Objekts zu sehen ist. Dabei ist es unerheblich in welcher Reihenfolge das erste Vergrößerungssystem und das zweite Vergrößerungssystem aufeinanderfolgen. Das Operationsmikroskop ist meist stereoskopisch, es ist also für jedes Auge ein eigener Beobachtungsstrahlengang vorgesehen.

Die Erfindung hat erkannt, dass die Einfügung eines zweiten Vergrößerungssystems zwischen der Frontlinse und dem Einblick möglich ist, ohne dass das Operationsmikroskop im Übrigen angepasst werden müsste. Da weitere Anpassungen nicht vorgenommen werden müssen, ist diese Lösung trotz des zusätzlich erforderlichen zweiten Vergrößerungssystems kostengünstig. Es bleibt zudem die hohe optische Qualität erhalten. Dies gilt insbesondere im Vergleich mit einem auf großen Dehnungsfaktor ausgelegten einzelnen Vergrößerungssystem. Versuche haben gezeigt, dass die optische Qualität abnimmt, wenn man versucht, den Dehnungsfaktor gegenüber den heute gängigen Vergrößerungssystemen zu erhöhen.

Durch die Hintereinanderschaltung zweier Vergrößerungssysteme erhält man einen Gesamtdehnungsfaktor der größer ist als der Dehnungsfaktor eines einzelnen Vergrößerungssystems. Wenn der Gesamtdehnungsfaktor groß ist, wird auch der mit dem Operationsmikroskop zugängliche Vergrößerungsbereich erweitert, wobei der Begriff Vergrößerungsbereich den Bereich zwischen dem kleinstmöglichen und dem größtmöglichen Vergrößerungsfaktor bezeichnet. Erweitert ist der Vergrößerungsbereich verglichen mit einem Operationsmikroskop, das nur das erste Vergrößerungssystem umfasst. Das zweite Vergrößerungssystem kann dazu verschiedene Zustände im Beobachtungsstrahlengang annehmen. In einer Ausführungsform hat das zweite Vergrößerungssystem einen festen Vergrößerungsfaktor. Der größere Gesamtdehnungsfaktor kann sich in diesem Fall daraus ergeben, dass das zweite Vergrößerungssystem in einem ersten Zustand im Beobachtungsstrahlengang angeordnet ist und in einem zweiten Zustand außerhalb des Beobachtungsstrahlengangs angeordnet ist. Um den Wechsel zwischen diesen Zuständen zu ermöglichen, kann das zweite Vergrößerungssystem beweglich an dem Operationsmikroskop aufgehängt sein. Alternativ kann das zweite Vergrößerungssystem kann als Modul ausgebildet sein, das von dem Operationsmikroskop getrennt werden kann.

Wenn das zweite Vergrößerungssystem einen festen Vergrößerungsfaktor hat, ist dieser vorzugsweise größer als 1, wobei 1 den Fall bezeichnet, dass keine Vergrößerung stattfindet. Andererseits ist der Vergrößerungsfaktor vorzugsweise kleiner als der Dehnungsfaktor des ersten Vergrößerungssystems. Der Vergrößerungsbereich, den das Operationsmikroskop bei Hintereinanderschaltung beider Vergrößerungssysteme hat, schließt sich dann nahtlos an den Vergrößerungsbereich an, der ohne das zweite Vergrößerungssystem gegeben ist. An der einen Grenze des Vergrößerungsbereichs ist die variable Vergrößerung des ersten Vergrößerungssystems am einen Anschlag und das zweite Vergrößerungssystem ist im Beobachtungsstrahlengang angeordnet. An der zweiten Grenze des Vergrößerungsbereichs ist die variable Vergrößerung des ersten Vergrößerungssystems am anderen Anschlag und das zweite Vergrößerungssystem ist außerhalb des Beobachtungsstrahlengangs angeordnet. Aus diesen beiden Grenzen des Vergrößerungsbereichs kann der Gesamtdehnungsfaktor ermittelt werden.

Das zweite Vergrößerungssystem kann alternativ einen variablen Vergrößerungsfaktor haben. Der Vergrößerungsfaktor des zweiten Vergrößerungssystems kann separat vom Vergrößerungsfaktor des ersten Vergrößerungssystems einstellbar sein. Komfortabler für den Benutzer ist es, wenn die Einstellung der Vergrößerungsfaktoren der beiden Vergrößerungssysteme miteinander gekoppelt ist. Die Koppelung ist vorzugsweise derart, dass der gesamte Vergrößerungsbereich von dem Zustand, in dem beide Vergrößerungssysteme den kleinsten Vergrößerungsfaktor haben, bis zu dem Zustand, in dem beide Vergrößerungssysteme den größten Vergrößerungsfaktor haben, kontinuierlich durchfahren werden kann. Der Gesamtdehnungsfaktor kann aus diesen beiden Grenzen ermittelt werden. Das Operationsmikroskop kann so eingerichtet sein, dass für die Einstellung beider Vergrößerungssysteme nur ein einzelnes Bedienelement vorgesehen ist. Die Vergrößerungssysteme können dazu mit Elektromotoren versehen sein, die über das Bedienelement betätigt werden.

Der variable Vergrößerungsfaktor eines Vergrößerungssystems kann auf unterschiedlichen Mechanismen beruhen. Das Vergrößerungssystem kann beispielsweise eine Mehrzahl von Unter-Vergrößerungssystemen umfassen, die feste, aber unterschiedliche Vergrößerungsfaktoren haben und die wahlweise in den Strahlengang eingebracht werden (Galilei-Wechsler). Möglich ist auch ein Vergrößerungssystem, bei dem der Vergrößerungsfaktor kontinuierlich einstellbar ist (Zoom-System) .

Der Gesamtdehnungsfaktor, den die Kombination aus dem ersten und dem zweiten Vergrößerungssystem bietet, ist vorzugweise größer als 10, weiter vorzugsweise größer als 20, weiter vorzugsweise größer als 30. Wenn der Gesamtdehnungsfaktor der Vergrößerungssysteme groß ist, kann mit dem Operationsmikroskop ein umfangreicher Vergrößerungsbereich abgedeckt werden.

Die Frontlinse kann eine Linse mit fester Brennweite sein. Durch die Frontlinse wird dann ein fester Arbeitsabstand definiert, der zwischen der Frontlinse und dem Objekt einzuhalten ist. Flexibler einsetzbar ist das Operationsmikroskop, wenn eine Frontlinse mit variabler Brennweite eingesetzt wird (Vario-Frontlinse). Der Arbeitsabstand zwischen der Frontlinse und dem Objekt kann dann je nach Bedarf unterschiedlich eingestellt werden. Mit Vario-Frontlinsen erreichen Operationsmikroskope allgemein nicht den Vergrößerungsfaktor, der bei Frontlinsen mit fester Brennweite erreicht wird. Die Anwendung der Erfindung in Kombination mit einer Vario-Frontlinse ist deswegen von besonderem Interesse.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erste Ausführungsform eines erfindungsgemä-ßen Operationsmikroskops;
- Fig. 2:: eine schematische Darstellung eines Vergröße-rungssystems mit variablem Vergrößerungsfaktor; und
- Fig. 3:: eine zweite Ausführungsform eines erfindungsgemä-ßen Operationsmikroskops.

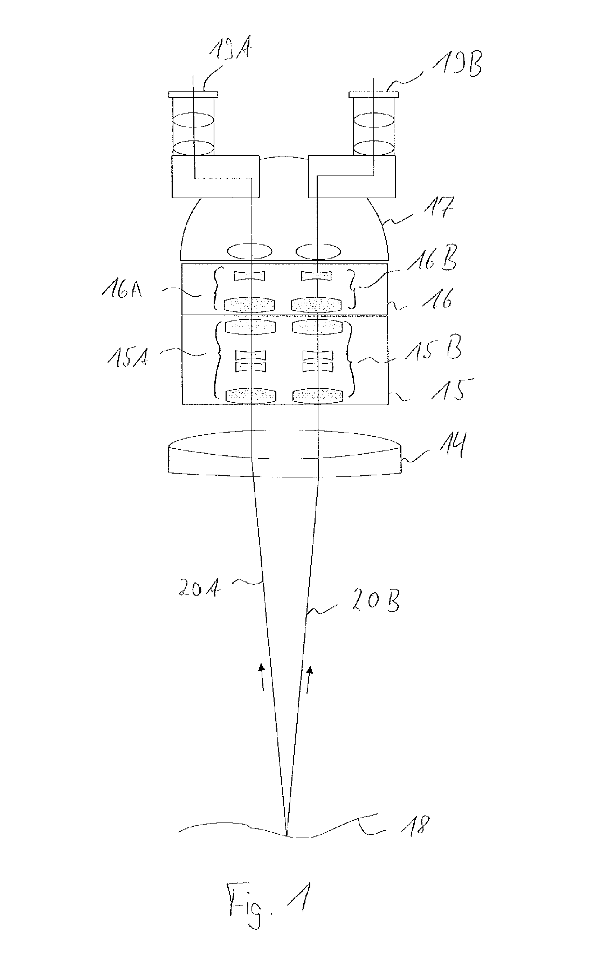

Eins stereoskopisches Operationsmikroskop in Fig. 1 umfasst eine Frontlinse 14, ein erstes Vergrößerungssystem 15, ein zweites Vergrößerungssystem 16 und einen Einblick 17 mit Okularen 19A und 19B. Diese Elemente sind so zueinander angeordnet, dass man beim Blick durch die Okulare 19A, 19B ein vergrößertes Bild eines vor der Frontlinse 14 angeordneten Objekts 18 sieht. Nach dem Durchtritt durch die gemeinsame Fronlinse 14 teilt sich der Beobachtungsstrahlengang in einen Strahlengang 20A für das eine Auge und einen Strahlengang 20B für das andere Auge auf. Das Operationsmikroskop ist also stereoskopisch.

Die Frontlinse 14 hat eine feste Brennweite. Der Arbeitsabstand zwischen der Frontlinse 14 und dem Objekt 18 entspricht der Brennweite der Frontlinse 14 und ist damit fest vorgegeben. Ein von einem Punkt des Objekts 18 ausgehender Strahlengang 20A, 20B wird durch die Frontlinse 14 in ein paralleles Strahlenbündel umgewandelt, das dann in das erste Vergrößerungssystem 15 eintritt. Die Funktionsweise eines solchen afokalen Vergrößerungssystems wird nachfolgend erläutert.

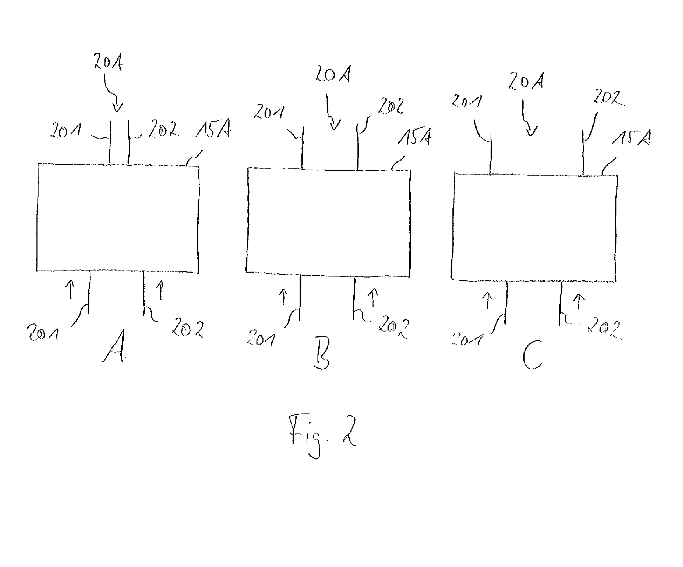

Das erste Vergrößerungssystem 15 umfasst ein linkes Vergrößerungssystem 15A für den Strahlengang 20A und ein rechtes Vergrößerungssystem 15B für den Strahlengang 20B. Die Funktionsweise eines Vergrößerungssystems wird anhand von Fig. 2 am Beispiel des linken Vergrößerungssystems 15A erläutert. Ein in das Vergrößerungssystem 15A eintretendes paralleles Strahlenbündel 20A tritt als paralleles Strahlenbündel 20A wieder aus. Zwei parallele Lichtstrahlen 201, 202 des Strahlenbündels 20A haben je nach eingestelltem Vergrößerungsfaktor des Vergrößerungssystems 15A beim Eintritt in das Vergrößerungssystem 15A einen anderen Abstand zueinander als beim Austritt.

In Fig. 2A ist der Vergrößerungsfaktor des Vergrößerungssystems 15A auf einen Wert kleiner 1 eingestellt. Der Abstand der beiden Lichtstrahlen 201, 202, deren Richtung durch Pfeile angedeutet ist, ist beim Eintritt in das Vergrößerungssystem 15A größer als beim Austritt. In Fig. 2B ist der Vergrößerungsfaktor gleich 1. In diesem Sonderfall findet keine Vergrößerung statt, sondern die Lichtstrahlen 201, 202 haben beim Eintritt und Austritt den gleichen Abstand zueinander. In Fig. 2C ist der Vergrößerungsfaktor größer als 1 und der Abstand zwischen den Lichtstrahlen 201, 202 ist beim Austritt größer als beim Eintritt.

Das rechte Vergrößerungssystem 15B funktioniert analog. Die Vergrößerungssysteme 15A, 15B sind so miteinander gekoppelt, dass der Vergrößerungsfaktor immer gemeinsam eingestellt wird. Es ist dazu ein in Fig. 1 nicht dargestelltes Bedienelement vorgesehen, über das mechanisch auf die optischen Elemente des Vergrößerungssystems 15 eingewirkt wird, um deren Abstand zueinander zu ändern. Der Vergrößerungsfaktor des Vergrößerungssystems 15 kann zwischen 1/√6 und √6 kontinuierlich variiert werden. Der als Quotient aus diesen beiden Werten ermittelte Dehnungsfaktor des Vergrößerungssystems 15 beträgt sechs.

Das an das erste Vergrößerungssystem 15 anschließende zweite Vergrößerungssystem 16 ist ebenfalls stereoskopisch (16A, 16B), hat aber einen festen Vergrößerungsfaktor im Bereich zwischen 1,5 und 3. Das zweite Vergrößerungssystem 16 ist als Modul ausgebildet, das wahlweise in den Strahlengang des Operationsmikroskops eingebracht werden bzw. aus diesem entfernt werden kann. Das Modul kann zu diesem Zweck beispielsweise schwenkbar mit dem Gehäuse des Operationsmikroskops verbunden sein. Möglich sind auch Ausführungsformen, bei denen das Modul vollständig von dem Operationsmikroskop getrennt wird. Da der feste Vergrößerungsfaktor des zweiten Vergrößerungssystems 16 kleiner ist als der Dehnungsfaktor des ersten Vergrößerungssystems 15, kann die Vergrößerung kontinuierlich eingestellt werden. Der Vergrößerungsbereich, der mit dem zweiten Vergrößerungssystem 16 zugänglich ist, schließt sich nahtlos an den Vergrößerungsbereich an, der ohne das zweite Vergrößerungssystem 16 zugänglich ist.

Der Einblick 17, der sich an das zweite Vergrößerungssystem 16 anschließt, hat eine Eigenvergrößerung von 8. Der Vergrößerungsfaktor des Operationsmikroskops insgesamt ergibt sich als Produkt aus den Vergrößerungsfaktoren der Vergrößerungssysteme 15, 16 und des Einblicks 17 sowie dem Quotienten aus 250mm/Brennweite Frontlinse. Der Wert 250 mm gibt dabei die Bezugssehweite an.

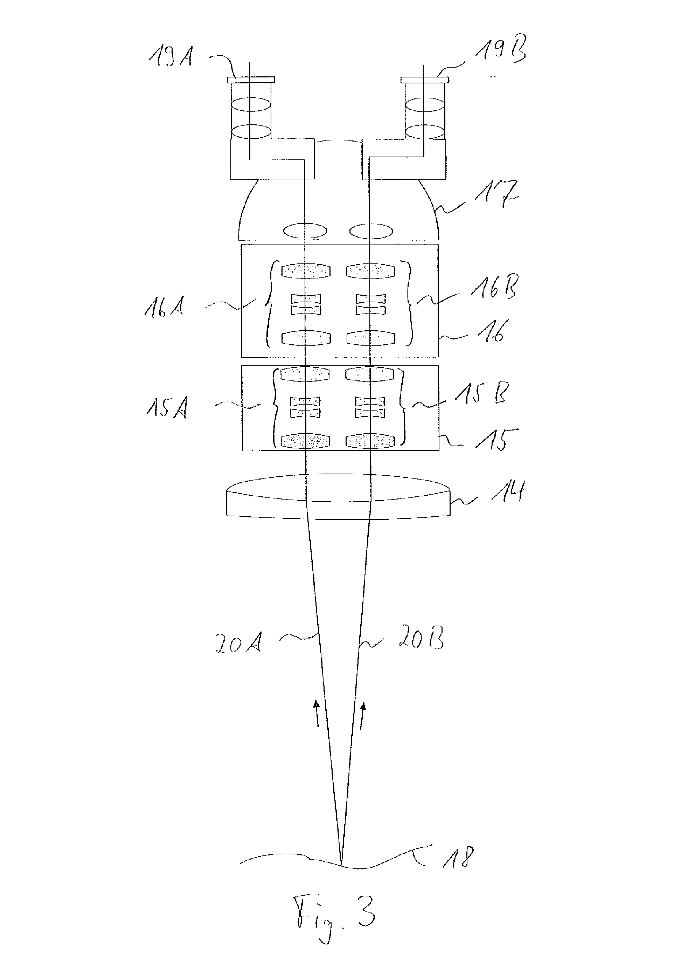

Die Ausführungsform gemäß Fig. 3 unterscheidet sich von Fig. 1 in zweierlei Hinsicht. Erstens ist die Frontlinse 14 eine Vario-Frontlinse, deren Brennweite je nach Bedarf variiert werden kann. Zweitens hat das zweite Vergrößerungssystem 16 einen variablen Vergrößerungsfaktor. Jedes der Vergrößerungssysteme 15, 16 ist mit einem nicht dargestellten Elektromotor ausgestattet, mit dem der Vergrößerungsfaktor eingestellt werden kann. Die Elektromotoren sind so miteinander gekoppelt, dass sie über ein gemeinsames Bedienelement bedient werden können. Mit dem Bedienelement werden beide Elektromotoren gleichzeitig bedient, so dass der gesamte Vergrößerungsbereich von dem kleinstmöglichen Vergrößerungsfaktor bis zum größtmöglichen Vergrößerungsfaktor kontinuierlich durchfahren werden kann.

## Patentansprüche

1. Operationsmikroskop mit einer Frontlinse (14), einem Einblick (17) und einem zwischen der Frontlinse (14) und dem Einblick (17) angeordneten ersten afokalen Vergrößerungssystem (15), wobei die Frontlinse (14), das erste Vergrößerungssystem (15) und der Einblick (17) einen Beobachtungsstrahlengang (20A, 20B) definieren und wobei das erste Vergrößerungssystem (15) einen variablen Vergrößerungsfaktor hat, **dadurch gekennzeichnet, dass** zwischen der Frontlinse (14) und dem Einblick (17) ein zweites afokales Vergrößerungssystem (16) vorgesehen ist.

2. Operationsmikroskop nach Anspruch 1, **dadurch gekennzeichnet, dass** das zweite Vergrößerungssystem (16) einen festen Vergrößerungsfaktor hat.

3. Operationsmikroskop nach Anspruch 1 oder 2, **dadurch gekennzeichnet, dass** das zweite Vergrößerungssystem (16) in einem ersten Zustand im Beobachtungsstrahlengang (20A, 20B) angeordnet ist und in einem zweiten Zustand außerhalb des Beobachtungsstrahlengangs (20A, 20B) angeordnet ist.

4. Operationsmikroskop nach Anspruch 3, **dadurch gekennzeichnet, dass** das zweite Vergrößerungssystem (16) beweglich an dem Operationsmikroskop aufgehängt ist.

5. Operationsmikroskop nach Anspruch 3, **dadurch gekennzeichnet, dass** das zweite Vergrößerungssystem (16) Bestandteil eines von dem Operationsmikroskop trennbaren Moduls ist.

6. Operationsmikroskop nach Anspruch 1, **dadurch gekennzeichnet, dass** das zweite Vergrößerungssystem (16) einen variablen Vergrößerungsfaktor hat.

7. Operationsmikroskop nach Anspruch 6, **dadurch gekennzeichnet, dass** der Vergrößerungsfaktor kontinuierlich variabel ist.

8. Operationsmikroskop nach Anspruch 6 oder 7, **dadurch gekennzeichnet, dass** das erste Vergrößerungssystem (15) und das zweite Vergrößerungssystem (16) Verstellmechanismen für den Vergrößerungsfaktor aufweisen und dass die Verstellmechanismen miteinander gekoppelt sind.

9. Operationsmikroskop nach einem der Ansprüche 6 bis 8, **dadurch gekennzeichnet, dass** der Vergrößerungsbereich zwischen dem kleinstmöglichen Vergrößerungsfaktor und dem größtmöglichen Vergrößerungsfaktor kontinuierlich durchfahren werden kann.

10. Operationsmikroskop nach einem der Ansprüche 1 bis 9, **dadurch gekennzeichnet, dass** der Gesamtdehnungsfaktor größer ist als 10, vorzugsweise größer ist als 20, weiter vorzugsweise größer ist als 30.

11. Operationsmikroskop nach einem der Ansprüche 1 bis 10, **dadurch gekennzeichnet, dass** die Frontlinse (14) eine Vario-Linse ist.
